# DEMANDE DE BREVET EUROPEEN

(11) **EP 1 177 768 A1**
(43) Date de publication de la demande: **06.02.2002**
(21) Numéro de dépôt: 01402083.8
(22) Date de dépôt: 01.08.2001
(51) Int. Cl.: A61B 8/08

(54) **Dispositif et procédé pour mesurer par effet doppler, l'évolution du diamètre d'une veine**

(30) Priorité: 03.08.2000 FR 0010238
(71) Demandeur: Etat-Francais représenté par le Délégué Général pour L'Armement, 94114 Arcueil Cèdex (FR)
(72) Inventeur: Caterini, Richard, Orlienas (FR); Trifot, Michel, 69740 Genas (FR)

(57) **Abrégé**

- L'invention concerne un dispositif pour mesurer par effet Doppler, l'évolution du diamètre d'une veine.
- Selon l'invention, le dispositif fonctionne de manière :
   - lors de la génération d'un premier signal, à commander une sonde d'émission et de réception (3) en mode continu, de manière à obtenir en fonction de la position de la sonde d'émission et de réception par rapport à la veine, un signal audible sur un haut-parleur adapté pour localiser la veine,
   - et lors de la génération d'un second signal qui intervient après la localisation de la veine, à commander la sonde d'émission et de réception (3) en mode discontinu et à déterminer, à partir des variations de fréquences entre les ondes ultrasonores émises et reçues, le diamètre de la veine pour assurer l'affichage de son évolution

## Description

La présente invention concerne le domaine technique de la mesure par effet Doppler, du diamètre d'une veine d'un patient.

L'objet de l'invention trouve une application particulièrement avantageuse dans le domaine du contrôle de la volémie sanguine chez un patient.

Dans le domaine technique ci-dessus, il doit être considéré que la prise en charge thérapeutique d'un choc hypo-volémique implique la réalisation rapide d'un remplissage vasculaire pour maintenir la volémie. La tendance des médecins est donc de perfuser rapidement le patient avec des liquides de remplissage. Il apparaît fréquemment une surcharge hydrique induite provoquant notamment une dilution des facteurs de coagulation qui augmentent les risques d'hémorragie et une infiltration des tissus avec création entre autre, d'oedèmes cérébraux et pulmonaires.

Il apparaît donc le besoin de pouvoir contrôler la variation de la volémie lors de cette phase thérapeutique délicate et essentielle pour un patient.

Compte tenu du fait que la volémie n'est pas actuellement mesurée de façon efficace, il a été envisagé d'apprécier au cours de l'opération de remplissage vasculaire, la variation de section de certaines veines ayant une capacité d'expansion, telles que la veine jugulaire ou fémorale. Ainsi, l'observation de l'évolution du diamètre d'une veine permet d'apprécier le niveau de remplissage vasculaire et, par suite, de déterminer le débit de remplissage.

Il apparaît donc le besoin de disposer d'un dispositif adapté pour déterminer l'évolution du diamètre d'une veine ayant une capacité d'expansion, afin d'apprécier le taux de remplissage volémique sanguin d'un patient placé dans des conditions environnementales souvent délicates, un tel dispositif étant conçu pour être autonome, de faible encombrement et d'une grande facilité d'utilisation.

L'objet de l'invention vise donc à satisfaire ce besoin en proposant un dispositif pour mesurer par effet Doppler, l'évolution du diamètre d'une veine.

Selon l'invention, le dispositif comporte dans un boîtier :
- au moins une sonde d'émission et de réception d'ondes ultrasonores, reliée à une unité de commande et de traitement,
- des moyens de traitement faisant partie de l'unité de commande et de traitement, et adaptés pour déterminer la variation de fréquence entre les ondes ultrasonores émises et reçues,
- un haut-parleur relié à la sortie des moyens de traitement,
- un moyen d'affichage relié à l'unité de commande et de traitement, et adapté pour visualiser l'évolution du diamètre de la veine,
- un moyen de génération d'un premier signal assurant le déclenchement d'une phase de fonctionnement de la sonde d'émission et de réception en mode continu,
- un moyen de génération d'un deuxième signal assurant le déclenchement d'une phase de fonctionnement de la sonde d'émission et de réception en mode discontinu,
- et une unité de commande et de traitement adaptée :
   - lors de la génération du premier signal, à commander la sonde d'émission et de réception en mode continu, de manière à obtenir en fonction de la position de la sonde d'émission et de réception par rapport à la veine, un signal audible sur le haut-parleur adapté pour localiser la veine,
   - lors de la génération du second signal qui intervient après la localisation de la veine, à commander la sonde d'émission et de réception en mode discontinu et à déterminer, à partir des variations de fréquences entre les ondes ultrasonores émises et reçues, le diamètre de la veine pour assurer l'affichage de son évolution,
- et une alimentation électrique pour les différents composants électriques du dispositif.

L'objet de l'invention vise également à proposer un nouveau procédé pour mesurer par effet Doppler, l'évolution du diamètre d'une veine chez un patient. Selon l'invention, le procédé consiste
- à appliquer sur la peau du patient, la sonde d'émission et de réception du dispositif,
- à commander le fonctionnement de la sonde d'émission et de réception en mode continu,
- à déplacer la sonde d'émission et de réception sur la peau du patient jusqu'à obtenir un signal audible correspondant à la localisation de la veine recherchée,
- à maintenir en position fixe, la sonde d'émission et de réception
- à commander le fonctionnement de la sonde d'émission et de réception en mode discontinu de manière à déterminer à partir des variations de fréquences entre les ondes ultrasonores émises et reçues, le diamètre de la veine,
- et à assurer l'affichage de l'évolution du diamètre de la veine.

Diverses autres caractéristiques ressortent de la description faite ci-dessous en référence aux dessins annexés qui montrent, à titre d'exemples non limitatifs, des formes de réalisation et de mise en oeuvre de l'objet de l'invention.
La **fig. 1** est un schéma-bloc fonctionnel du dispositif de mesure conforme à l'invention.
La **fig. 2** est une vue schématique partielle d'un exemple de réalisation d'un dispositif conforme à l'invention.
La **fig. 3** est un schéma illustrant une caractéristique avantageuse de l'objet de l'invention.

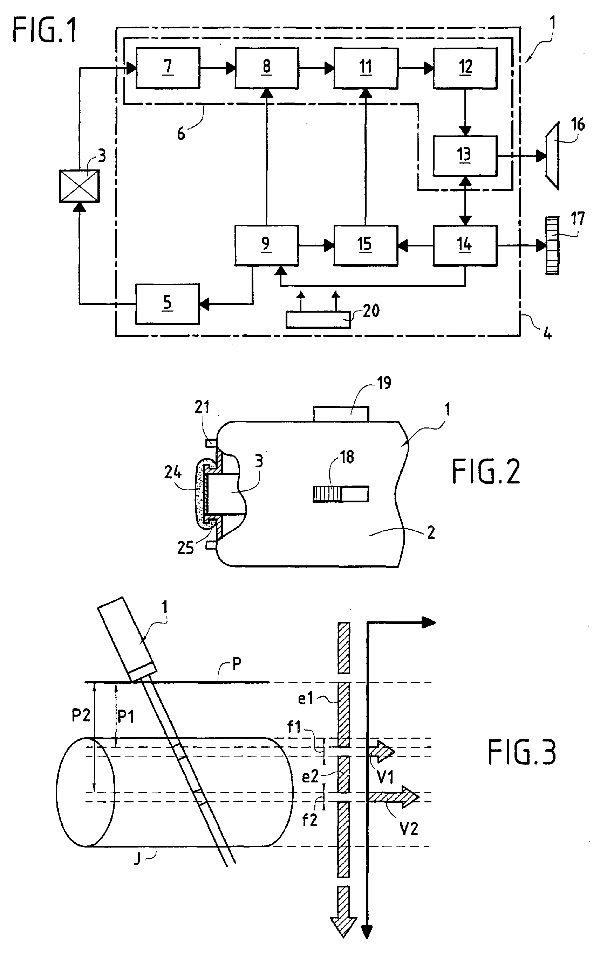

Tel que cela apparaît plus précisément aux **fig. 1** à **3,** l'objet de l'invention concerne un dispositif **1** adapté pour mesurer par effet Doppler, l'évolution du diamètre d'une veine **J** schématisée à la **fig. 3.** Le dispositif **1** selon l'invention se présente sous la forme d'un boîtier **2** de faibles dimensions susceptible de pouvoir être manipulé, en vue d'être positionné sur la peau **p** d'un patient en dessous de laquelle est située une veine **J** présentant une capacité d'expansion, telle que la veine fémorale ou jugulaire.

Le dispositif **1** comporte une sonde **3** d'émission et de réception d'ondes ultrasonores, reliée à une unité de commande et de traitement **4**. De façon plus précise, l'unité de commande et de traitement **4** comporte un étage émetteur **5** délivrant à la sonde d'émission et de réception **3,** un signal électrique sinusoïdal pour assurer l'émission d'ondes ultrasonores. Par exemple, la sonde d'émission et de réception **3** comporte une céramique émettrice transformant par effet piézo-électrique, le signal électrique reçu de l'étage émetteur **5** en un phénomène mécanique de même fréquence. Cette céramique émettrice produit ainsi une vibration mécanique de fréquence élevée entraînant la génération d'une onde ultrasonore se propageant dans une direction privilégiée perpendiculaire à la surface de la céramique émettrice. Un tel faisceau ultrasonore se propage dans les tissus jusqu'à la rencontre de la veine **J** dans laquelle les éléments figurés du sang circulant font office de réflecteur. Les ondes réfléchies sont ainsi reçues par la sonde d'émission et de réception **3** qui comporte également une céramique de réception qui par effet piézo-électrique inverse, transforme les ondes ultrasonores reçues en un signal électrique de même fréquence.

L'unité de commande et de traitement **4** comporte des moyens de traitement **6** adaptés pour déterminer la variation de fréquence entre les ondes ultrasonores émises et les ondes ultrasonores reçues. Les moyens de traitement **6** comportent un étage d'amplification **7** des signaux reçus par la sonde d'émission et de réception **3**. La sortie de l'étage d'amplification **7** est reliée à un circuit de démodulation synchrone **8** commandé par un pilote logique **9** qui assure le contrôle de l'étage émetteur **5**. La sortie du circuit de démodulation **8** est relié par l'intermédiaire d'un circuit échantillonneur **11,** à un étage de filtrage **12.** La sortie de l'étage de filtrage **12** est reliée à un circuit de traitement **13** adapté pour déterminer la variation de fréquence entre les signaux émis et reçus. Le circuit de traitement **13** est relié à un microprocesseur central de commande **14** qui assure aussi, notamment, le contrôle du pilote logique **9**, et d'un circuit de génération **15** d'une fenêtre de mesure, dont la fonction apparaîtra plus précisément dans la suite de la description.

Le dispositif **1** selon l'invention comporte également un haut-parleur **16** relié à la sortie des moyens de traitement **6** et un moyen d'affichage **17** relié au microprocesseur **14** et adapté pour visualiser, par exemple, l'évolution du diamètre de la veine **J**.

Le dispositif de mesure **1** selon l'invention comporte également un moyen **18** de génération d'un premier signal assurant le déclenchement d'une phase de fonctionnement de la sonde d'émission et de réception **3** selon un mode continu. Par exemple, un tel moyen de génération **18** est constitué par un interrupteur de mise sous tension du dispositif **1**.

Le dispositif **1** selon l'invention comporte également un moyen **19** de génération d'un deuxième signal assurant le déclenchement d'une phase de fonctionnement de la sonde d'émission et de réception **3** selon un mode discontinu. Par exemple, le moyen **19** de génération d'un deuxième signal peut être constitué par l'intermédiaire d'un bouton poussoir accessible facilement par l'utilisateur. Le dispositif de mesure **1** selon l'invention comporte également une alimentation électrique **20** pour les différents composants électriques ou électroniques montés à l'intérieur du boîtier **2**. L'unité de contrôle et de traitement **4** comporte également des moyens de programmation permettant de mettre en oeuvre le procédé de mesure tel que décrit ci-après.

Lors de la génération du premier signal par l'interrupteur de mise sous tension **18**, l'unité de commande et de traitement **4** commande la sonde d'émission et de réception **3** en mode continu. En d'autres termes, la sonde d'émission et de réception émet de manière continue des ondes ultrasonores avec une fréquence comprise entre 3 et 7 MHz et, de préférence de l'ordre de 4 MHz. Le dispositif **1** est positionné sur la peau **P** du patient, en vue de localiser la veine **J** recherchée. Les moyens de traitement **6** assurent la détermination de la variation de fréquence entre les ondes ultrasonores émises et les ondes ultrasonores reçues. La variation de fréquence est transmise au haut-parleur **16** qui fournit un signal sonore permettant de localiser la veine **J.** En effet, il doit être considéré que le signal fourni par le haut-parleur **16** permet de différencier un écoulement artériel bruyant et de haute fréquence, car rapide et important, d'un écoulement sanguin à l'intérieur d'une veine, caractérisé par un bruit sourd et moins puissant. Le dispositif **1** est ainsi déplacé jusqu'à obtenir un son sur le haut-parleur **16** caractéristique de la présence d'une veine **J**. Le signal audible délivré par le haut-parleur **16** sert ainsi à l'orientation et au positionnement du dispositif 1 par rapport à la veine **J** recherchée.

Lorsque la veine **J** est localisée, le dispositif **1** est maintenu en place sur la peau du patient. Il est à noter que le dispositif **1** peut comporter un système de marquage **21** monté à une distance déterminée de la sonde d'émission et de réception **3,** afin de permettre de repérer sur la peau du patient, l'endroit de la sonde d'émission et de réception correspondant à la localisation de la veine **J**. Par exemple, un tel système de marquage **21** peut être constitué par un système d'écriture inscrivant par exemple deux points sur la peau, au milieu desquels est centrée la sonde d'émission et de réception **3**.

Lorsque la localisation de la veine **J** est effectuée, l'utilisateur assure par le moyen de génération **19** d'un deuxième signal, le déclenchement d'une seconde phase de fonctionnement de la sonde d'émission et de réception **3**, selon un mode discontinu. Lors de cette phase, l'unité de commande et de traitement **4** permet de déterminer, à partir des variations de fréquences entre les ondes ultrasonores émises et reçues, le diamètre de la veine pour assurer l'affichage de son évolution. La sonde d'émission et de réception **3** émet ainsi, de manière discontinue, des ondes ultrasonores présentant une fréquence comprise entre 3 et 7 MHz et, de préférence, de l'ordre de 4 MHz.

A cet effet, l'unité de commande et de traitement **4** pilote la sonde d'émission et de réception **3** afin d'assurer l'émission successive d'impulsions d'ondes ultrasonores **e**_{**1**}**, e**_{**2**}**, e**_{**3**}**, ..., e**_{**i**} séparées chacune par une fenêtre de mesure respectivement **f**_{**1**}**, f**_{**2**}**, f**_{**3**}**, ..., f**_{**i**} des ondes ultrasonores reçues. Durant l'intervalle de temps séparant deux émissions successives d'impulsions **e**_{**i**}**,** il est procédé à une détermination de la vitesse d'écoulement **V**_{**i**}**.** Tel que cela apparaît dans l'exemple illustré à la **fig. 3,** après l'émission de chaque impulsion **e**_{**1**}**, e**_{**2**}**,** c'est-à-dire pendant la fenêtre de mesure respectivement **f**_{**1**}**, f**_{**2**}**,** il est procédé à la détermination d'une vitesse respectivement **V**_{**1**}**, V**_{**2**} correspondant aux éléments réflecteurs contenus dans la veine **J** et situés à un niveau respectivement **P**_{**1**}**, P**_{**2**} par rapport à la peau **P** du patient.

Selon une caractéristique préférée de réalisation, la fréquence d'émission des impulsions d'ondes ultrasonores **e**ᵢ présente un caractère variable pour assurer la scrutation complète d'une section de la veine **J.** A cet effet, le microprocesseur **14** commande le circuit de génération **15** d'une fenêtre de mesure **f**_{**i**}, de manière à assurer le décalage temporel des fenêtres de mesure qui intervient entre chaque impulsion de fréquence variable. Il peut ainsi être déterminé la courbe des vitesses **V** sur une section donnée de la veine **J** permettant de repérer les parois de la veine. Il peut ainsi être déterminé la courbe des vitesses du débit sanguin sur une section de la veine. A partir de l'incidence du positionnement du dispositif **1** par rapport à l'axe de la veine **J**, il peut être apprécié le diamètre réel de la veine **J**. Les valeurs du diamètre de la veine J sont ainsi affichées sur le moyen d'affichage **17** permettant d'apprécier l'évolution du diamètre de la veine **J**. Par exemple, le moyen d'affichage **17** peut être formé par un bargraph.

Selon une caractéristique préférée de réalisation, le dispositif **1** comporte une membrane d'adaptation acoustique **24**, dont l'impédance est adaptée aux ondes ultrasonores, afin de favoriser l'adaptation acoustique entre l'électronique de transduction ultrasonore et la peau du patient. Cette membrane est montée en regard de la sonde d'émission et de réception **3**. Cette membrane d'adaptation acoustique **24** comporte des moyens de montage amovibles sur le boîtier pour permettre son retrait après utilisation. A cet effet, cette membrane **24** peut être constituée sous la forme d'un bonnet venant s'encastrer dans une rainure annulaire **25** aménagée sur le boîtier **2**, de manière que le bonnet se trouve monté en regard de la sonde d'émission et de réception **3**. Une telle membrane, qui peut être constituée à partir de silicone ou d'un coussin en feutre mince imbibé de gel acoustique injecté sous pression, présente la particularité de n'atténuer que très peu les ultrasons. Pendant la phase de stockage, une telle membrane peut être recouverte d'une pellicule de protection susceptible d'être retirée avant son montage sur le boîtier **2**.

## Revendications

1. Dispositif pour mesurer par effet Doppler, l'évolution du diamètre d'une veine **(J), caractérisé en ce qu'**il comporte dans un boîtier **(2)** :
- au moins une sonde d'émission et de réception d'ondes ultrasonores (**3**), reliée à une unité de commande et de traitement (**4**),
- des moyens de traitement (**6**) faisant partie de l'unité de commande et de traitement (**4**), et adaptés pour déterminer la variation de fréquence entre les ondes ultrasonores émises et reçues,
- un haut-parleur (**16**) relié à la sortie des moyens de traitement,
- un moyen d'affichage (**17**) relié à l'unité de commande et de traitement, et adapté pour visualiser l'évolution du diamètre de la veine,
- un moyen (**18**) de génération d'un premier signal assurant le déclenchement d'une phase de fonctionnement de la sonde d'émission et de réception en mode continu,
- un moyen (**19**) de génération d'un deuxième signal assurant le déclenchement d'une phase de fonctionnement de la sonde d'émission et de réception en mode discontinu,
- et une unité de commande et de traitement (**4**) adaptée
• lors de la génération du premier signal, à commander la sonde d'émission et de réception (**3**) en mode continu, de manière à obtenir en fonction de la position de la sonde d'émission et de réception par rapport à la veine (**J**), un signal audible sur le haut-parleur adapté pour localiser la veine **(J),**
• lors de la génération du second signal qui intervient après la localisation de la veine, à commander la sonde d'émission et de réception (**3**) en mode discontinu et à déterminer, à partir des variations de fréquences entre les ondes ultrasonores émises et reçues, le diamètre de la veine pour assurer l'affichage de son évolution,
- et une alimentation électrique (**20**) pour les différents composants électriques du dispositif.

2. Dispositif selon la revendication 1, **caractérisé en ce que** l'unité de commande et de traitement **(4)** pilote, en mode discontinu, la sonde d'émission et de réception (**3**), afin d'assurer l'émission successive d'impulsions d'ondes ultrasonores **(e**_{**i**}**)** séparées chacune par une fenêtre de mesure (**f**_{**i**}) des ondes ultrasonores reçues afin de déterminer la variation de la fréquence, la fréquence d'émission des impulsions étant variable pour assurer la scrutation du profil de la veine.

3. Dispositif selon la revendication 1, **caractérisé en ce que** l'unité de commande et de traitement (**4**) commande la sonde d'émission et de réception (**3**) en mode continu, pour assurer l'émission d'ondes ultrasonores dont la fréquence est comprise entre 3 et 7 MHz.

4. Dispositif selon la revendication 1, **caractérisé en ce que** l'unité de commande et de traitement commande la sonde d'émission et de réception (**3**) en mode discontinu, pour assurer l'émission d'ondes ultrasonores dont la fréquence est comprise entre 3 et 7 MHz.

5. Dispositif selon la revendication 1, **caractérisé en ce que** l'unité de commande et de traitement **(4)** comporte :
- un étage émetteur (**5**) délivrant à la sonde d'émission et de réception (**3**), un signal électrique sinusoïdal pour assurer l'émission d'ondes ultrasonores,
- et des moyens de traitement (**6**) comprenant un étage d'amplification (**7**) des signaux reçus par la sonde d'émission et de réception (**3**) relié à un circuit de démodulation synchrone (**8**) piloté par le signal électrique délivré par l'étage émetteur, la sortie du circuit de démodulation synchrone étant reliée par l'intermédiaire d'un circuit échantillonneur (**11**) et d'un étage de filtrage (**12**), à un circuit de traitement (**13**) adapté pour déterminer la variation de fréquence entre les signaux émis et reçus.

6. Dispositif selon l'une des revendications 1 à 5, **caractérisé en ce qu'**il comporte une membrane d'adaptation acoustique (**24**) montée en regard de la sonde d'émission et de réception (**3**).

7. Dispositif selon la revendication 6, **caractérisé en ce que** la membrane compressible d'adaptation acoustique **(24)** comporte des moyens de montage amovible sur le boîtier pour permettre son retrait après utilisation.

8. Dispositif selon l'une des revendications 1 à 7, **caractérisé en ce qu'**il comporte un système de marquage (**21**) monté à une distance déterminée de la sonde d'émission et de réception (**3**), afin de permettre de repérer sur la peau d'un patient, la position de la sonde d'émission et de réception.

9. Dispositif selon la revendication 1, **caractérisé en ce que** le moyen de génération du premier signal (**18**) est constitué par un bouton marche - arrêt, tandis que le moyen de génération du deuxième signal (**19**) est constitué par un interrupteur commandé.

10. Procédé pour mesurer par effet Doppler, l'évolution du diamètre d'une veine (**J** ) chez un patient, **caractérisé en ce qu'**il consiste :
- à appliquer sur la peau (**P**) du patient, la sonde d'émission et de réception (**3**) du dispositif conforme à l'une des revendications 1 à 9,
- à commander le fonctionnement de la sonde d'émission et de réception (**3**) en mode continu,
- à déplacer la sonde d'émission et de réception (**3**) sur la peau (**P**) du patient jusqu'à obtenir un signal audible correspondant à la localisation de la veine recherchée,
- à maintenir en position fixe, la sonde d'émission et de réception (**3**),
- à commander le fonctionnement de la sonde d'émission et de réception (**3**) en mode discontinu de manière à déterminer à partir des variations de fréquences entre les ondes ultrasonores émises et reçues, le diamètre de la veine,
- et à assurer l'affichage de l'évolution du diamètre de la veine (**J**).
